(19) Europäisches Patentamt
European Patent Office
Office européen des brevets

(11) Numéro de publication: **0 368 756 A1**

(12) **DEMANDE DE BREVET EUROPEEN**

(21) Numéro de dépôt: **89403081.6**

(51) Int. Cl.[5]: **C07D 451/02, A61K 31/46**

(22) Date de dépôt: **08.11.89**

Revendications pour les Etats contractants suivants: ES + GR.

(30) Priorité: **09.11.88 FR 8814608**

(43) Date de publication de la demande: **16.05.90 Bulletin 90/20**

(84) Etats contractants désignés: **AT BE CH DE ES FR GB GR IT LI LU NL SE**

(71) Demandeur: **ROUSSEL-UCLAF**
**35, boulevard des Invalides**
**F-75007 Paris(FR)**

(72) Inventeur: **Frechet, Daniel**
**45, rue Lecourbe**
**F-75015 Paris(FR)**
Inventeur: **Hunt, Peter Francis**
**18, rue de l'Hôtel Dieu**
**F-95500 Gonesse(FR)**
Inventeur: **Nedelec, Lucien**
**45, Boulevard de l'Ouest**
**F-93340 Le Raincy(FR)**
Inventeur: **Oberlander, Claude**
**2, rue Paul Albert**
**F-75018 Paris(FR)**

(74) Mandataire: **Tonnellier, Marie-José et al**
**111, route de Noisy B.P. no 9**
**F-93230 Romainville(FR)**

(54) **Nouveaux dérivés du dibenzocycloheptène, leur procédé de préparation et leur application comme médicaments.**

(57) L'invention a pour objet les composés de formule (I),

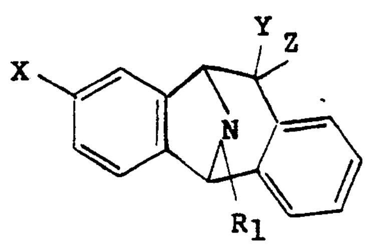

(I)

dans laquelle :
- X représente un groupe nitro ou un groupe $R_2R_3N$- dans lequel $R_2$ et $R_3$ identiques ou différents représentent un atome d'hydrogène, un radical alkyle, alkényle ou alkynyle, un radical $R_4CO$-, $-COOR_5$ ou $R_6R_7NCO$-, $R_4$, $R_5$, $R_6$ et $R_7$ représentent radical alkyle, $R_2$ et $R_3$ ne pouvant représenter tous deux un radical alkyle,
- Y et Z - ou bien représentent chacun un atome d'hydrogène
- ou bien forment ensemble avec l'atome de carbone auquel ils sont liés un groupe carbonyle et
- $R_1$ représente un atome d'hydrogène, un radical alkyle, alkényle ou alkynyle ou un radical $-COOR_8$ dans lequel $R_8$ représente un radical alkyle.

Les composés de formule (I) présentent d'intéressantes propriétés pharmacologiques qui justifient leur utilisation comme médicaments.

EP 0 368 756 A1

## Nouveaux dérivés du dibenzocycloheptène, leur procédé de préparation et leur application comme médicaments.

La présente invention concerne de nouveaux dérivés du dibenzocycloheptène, leur procédé de préparation et leur application comme médicaments.

L'invention a pour objet les composés de formule (I) :

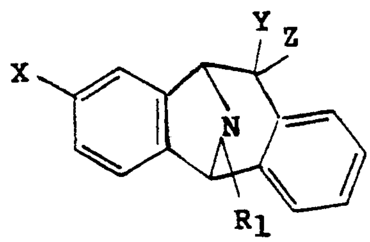

(I)

dans laquelle :

- X représente un groupe nitro ou un groupe $R_2R_3N$- dans lequel $R_2$ représente un atome d'hydrogène, un radical alkyle, alkényle ou alkynyle renfermant jusqu'à 8 atomes de carbone et $R_3$ représente un atome d'hydrogène, un radical alkyle, alkényle ou alkynyle renfermant jusqu'à 8 atomes de carbone, un radical arylalkyle renfermant jusqu'à 14 atomes de carbone, un radical $R_4\overset{O}{\overset{\|}{C}}$ - dans lequel $R_4$ représente un atome d'hydrogène ou un radical alkyle renfermant jusqu'à 8 atomes de carbone, un radical $-COOR_5$ dans lequel $R_5$ représente un radical alkyle renfermant jusqu'à 8 atomes de carbone, ou un radical $R_6R_7N\overset{O}{\overset{\|}{C}}$ -dans lequel $R_6$ et $R_7$ identiques ou différents, représentent un atome d'hydrogène ou un radical alkyle renfermant jusqu'à 8 atomes de carbone, à la condition que $R_2$ et $R_3$ ne représentent pas chacun un radical alkyle,
- Y et Z, ou bien représentent chacun un atome d'hydrogène,
ou bien forment ensemble avec l'atome de carbone auquel ils sont liés un groupe carbonyle et
- $R_1$ représente un atome d'hydrogène, un radical alkyle, alkényle ou alkynyle renfermant jusqu'à 8 atomes de carbone ou un radical $-COOR_8$ dans lequel $R_8$ représente un radical alkyle renfermant jusqu'à 8 atomes de carbone, ou un radical arylalkyle, éventuellement substitué, renfermant jusqu'à 14 atomes de carbone ainsi que leurs sels d'addition avec les acides organiques ou minéraux.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléïque, fumarique, succinique, tartrique, citrique, oxalique, glyoxylique, aspartique, alcanesulfoniques tels que les acides méthane ou éthanesulfoniques, arènesulfoniques, tels que les acides benzène ou para-toluènesulfoniques et arylcarboxyliques. Les produits de formule (I) peuvent exister sous forme racémique ou dédoublée. Lorsque l'un des radicaux mentionnés dans la définition des produits de formule (I) est un radical alkyle, il s'agit de préférence du radical méthyle, éthyle, propyle, isopropyle ou butyle.

Lorsque l'un des radicaux mentionnés dans la définition des produits de formule (I) est un radical alkényle, il s'agit de préférence d'un radical allyle.

Lorsque l'un des radicaux mentionnés dans la définition des produits de formule (I) est un radical alkynyle, il s'agit de préférence du radical propargyle.

Lorsque $R_1$ représente un radical arylalkyle, il s'agit de préférence du radical benzyle, éventuellement substitué par un atome d'halogène, un radical alkyle ou alkyloxy renfermant jusqu'à 4 atomes de carbone.

Parmi les composés préférés de l'invention, on peut citer :

- les composés de formule (I) dans lesquels X représente un groupe amino,
- les composés de formule (I) dans lesquels Y et Z représentent chacun un atome d'hydrogène,
- les composés de formule (I) dans lesquels $R_1$ représente un atome d'hydrogène, ainsi que leurs sels d'addition avec les acides organiques ou minéraux.

L'invention a tout particulièrement pour objet les produits dont la préparation est donnée ci-après dans la partie expérimentale, et notamment le produit de l'exemple 7.

Les composés de formule (I) et leurs sels d'addition avec les acides organiques ou minéraux présentent d'intéressantes propriétés pharmacologiques, ils antagonisent notamment l'effet neurotoxique des acides aminés excitateurs, ils présentent également des propriétés anti-convulsivantes.

Les composés de formule (I) et leurs sels d'addition avec les acides organiques ou minéraux pharmaceutiquement acceptables peuvent donc être utilisés comme médicaments utiles notamment pour prévenir les lésions cérébrales consécutives à un arrêt cardiaque, un accident vasculaire cérébral, une hypoglycémie ou une épilepsie.

La posologie utile peut être comprise entre 5 et 50 mg par jour par voie injectable ou orale par exemple.

La présente demande a donc également pour objet l'application, à titre de médicaments, des produits de formule (I) ainsi que leurs sels d'addition avec les acides pharmaceutiquement acceptables.

Parmi les médicaments préférés de l'invention on peut citer ceux qui sont constitués par les produits décrits en exemple notamment le produit de l'exemple 7, ainsi que leurs sels d'addition avec les acides pharmaceutiquement acceptables.

L'invention a enfin pour objet les compositions pharmaceutiques qui renferment au moins un dérivé précité ou l'un de ses sels d'addition avec les acides pharmaceutiquement acceptables, à titre de principe actif.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I) caractérisé en ce que l'on soumet un composé de formule (II) :

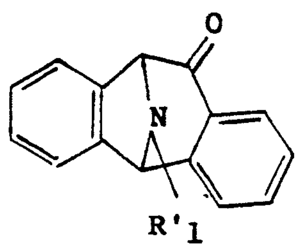

(II)

dans laquelle $R'_1$ représente un radical alkyle, alkényle ou alkynyle renfermant jusqu'à 8 atomes de carbone à l'action d'un agent de nitration pour obtenir le composé de formule $(I'_A)$ correspondant :

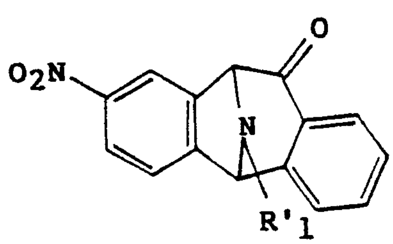

$(I'_A)$

puis soumet le composé de formule $(I'_A)$ ainsi obtenu à la totalité ou à une partie seulement des opérations suivantes dans un ordre quelconque :

- réduction sélective de la fonction nitro et éventuellement dans ce cas fonctionnalisation du radical amino obtenu,
- réduction sélective du groupe carbonyle,
- remplacement du radical $R'_1$ par un radical $-COOR_8$ et éventuellement dans ce cas, clivage du groupe $COOR_8$ obtenu condui sant au composé dans lequel $R'_1$ est un atome d'hydrogène,
- salification des produits de formule (I) ainsi obtenus.

Les composés de formule (II) utilisés comme produits de départ sont des produits connus qui peuvent être préparés selon le procédé décrit dans le brevet français 2170862. Dans un mode de réalisation préféré du procédé de l'invention :

- l'agent de nitration est l'acide nitrique utilisé seul ou en présence d'acide sulfurique,
- l'agent de réduction sélective du radical nitro est de préférence le chlorure stanneux en présence d'acide chlorhydrique concentré,
- l'agent de réduction sélective du groupe carbonyle est l'hydrate d'hydrazine en présence d'un hydroxyde de métal alcalin selon la méthode de Wolff-Kishner,
- le remplacement du radical $R'_1$ par le radical $-COOR_8$ est réalisé de préférence par action d'un halogènoformiate d'alkyle de formule :

$HalCOOR_8$

dans laquelle $R_8$ conserve sa signification précédente et Hal représente un atome d'halogène et de préférence par action d'un chloroformiate d'alkyle,

- le clivage du groupe $-COOR_8$ pour obtenir le groupe -NH- est réalisé de préférence par hydrolyse effectuée en milieu basique par exemple en présence d'un hydroxyde de métal alcalin,
- la salification est réalisée par action d'un acide.

L'invention a plus précisément pour objet un procédé de préparation caractérisé en ce que l'on soumet le composé de formule ($I_A$) :

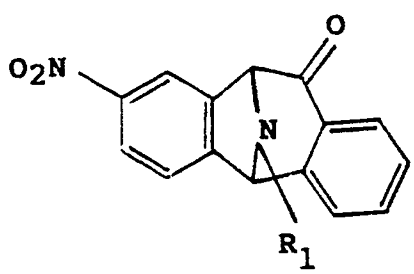

($I_A$)

dans laquelle $R_1$ conserve sa signification prédécente, à l'action d'un agent de réduction sélective du radical nitro, pour obtenir le composé de formule ($I_B$) correspondant :

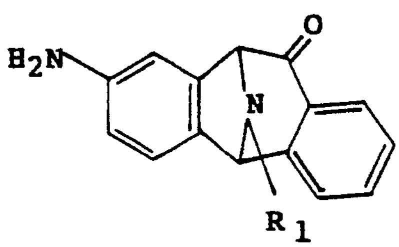

($I_B$)

que l'on soumet si désiré à l'action d'un agent de réduction sélective de la fonction cétone, pour obtenir le composé de formule ($I_C$) :

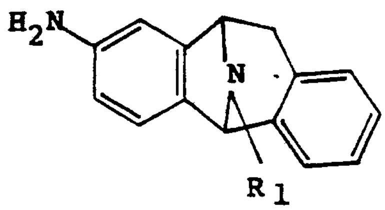

($I_C$)

puis si désiré, soumet les composés de formule (I) ainsi obtenus à l'action d'un acide pour en former les sels. L'invention a également pour objet un procédé caractérisé en ce que l'on soumet le composé de formule ($I''_A$) :

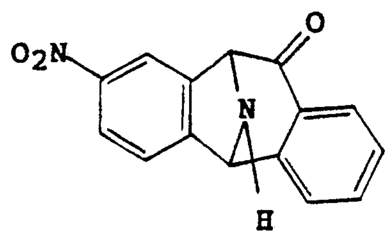

(I"A)

c'est-à-dire le composé de formule ($I_A$) dans lequel $R_1$ est un atome d'hydrogène,
ou bien à l'action d'un agent capable d'introduire le radical $R''_1$ pour obtenir le composé de formule ($I'_B$) :

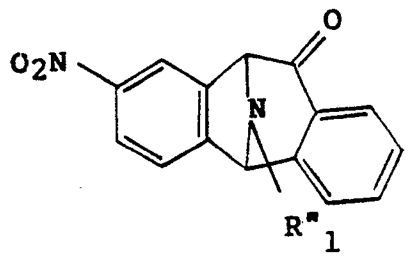

(I'B)

dans lequel $R''_1$ représente un radical arylalkyle éventuellement substitué, renfermant jusqu'à 14 atomes de carbone ou un radical $-COOR_8$, $R_8$ conservant sa signification précédente, que l'on soumet, le cas échéant, à l'action d'un agent de réduction du groupement carbonyle, pour obtenir le composé de formule ($I'_C$) :

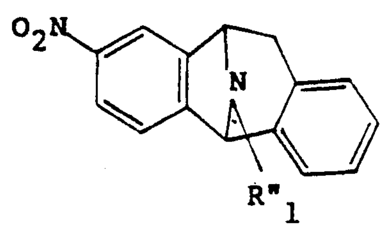

(I'C)

ou bien à l'action d'un agent de réduction du groupement carbonyle pour obtenir le composé de formule générale ($I''_B$) :

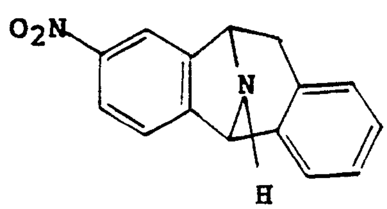

(I"B)

que l'on soumet, le cas échéant, à l'action d'un agent capable d'introduire le radical $R''_1$, pour obtenir le composé de formule ($I'_C$) :

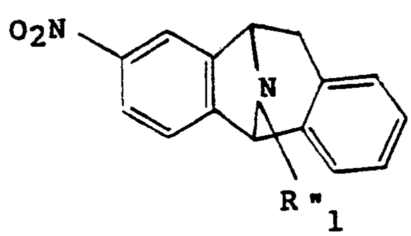

(I'C)

puis soumet, le cas échéant, le composé de formule ($I'_C$) ainsi obtenu, à l'action d'un agent de réduction du groupement nitro, pour obtenir le composé de formule ($I'_D$) :

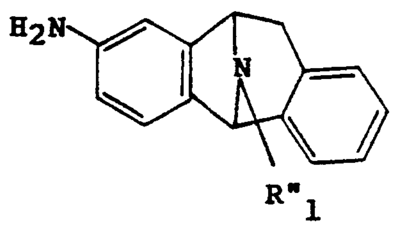

($I'_D$)

que l'on soumet, le cas échéant, à l'action d'un agent de fonctionnalisation du groupement amino pour obtenir le composé de formule ($I'_E$) :

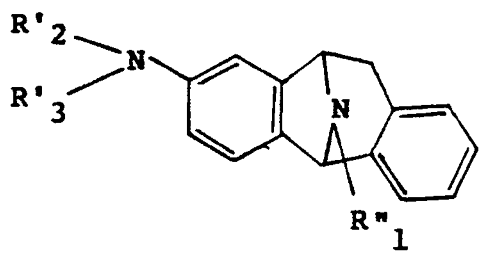

($I'_E$)

dans lequel $R'_2$ et $R'_3$ ayant la même signification que $R_2$ et $R_3$ sans pouvoir représenter tous deux un atome d'hydrogène, que l'on soumet, le cas échéant, à l'action d'un agent de déprotection de l'amine $-NR''_1$ pour obtenir le composé de formule ($I'_F$) :

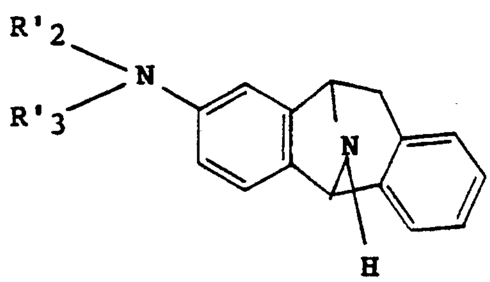

($I'_F$)

puis si désiré, soumet les composés de formule (I) ainsi obtenus à l'action d'un acide pour en former les sels.

Dans un mode de réalisation préféré :

- l'agent capable d'introduire le radical $R''_1$ est un dérivé de formule $HalCOOR_8$ ou un dérivé de formule $ArCH_2Hal$, Hal représentant un atome d'halogène, et Ar un radical aryle renfermant jusqu'à 14 atomes de carbone. Le radical $R''_1$ est de préférence un radical terbutoxycarbonyle ou benzyle.
- la déprotection de l'amine $NR''_1$ est réalisée comme indiquée ci-dessus lorsque $R''_1$ représente un radical $-COOR_8$, et selon les méthodes classiques de débenzylation par exemple par hydrogénation catalytique lorsque $R''_1$ est un radical arylalkyle.
- les autres étapes du procédé sont réalisées selon les modes de réalisation préférées déjà indiquées ci-dessus. Les exemples suivants illustrent l'invention sans toutefois la limiter.

**EXEMPLE 1 : 12-méthyl 8-nitro 10,11-dihydro 5,10-imino 5H-dibenzo[a,d]cycloheptène-11-one.**

On introduit sous agitation et atmosphère d'azote dans 850 ml d'une solution d'acide sulfurique 66° Bé, 170 g de 12-méthyl 10,11-dihydro 5,10-imino 5H-dibenzo[a,d]cycloheptène-11-one préparé selon le procédé décrit à l'exemple 15 du brevet français 2170862. On refroidit à -5° C et ajoute en environ 15 minutes à -5° C ± 2° C sous agitation et atmosphère d'azote 36 $cm^3$ d'une solution d'acide nitrique 48° Bé. On

maintient le mélange réactionnel pendant 1 heure à -5° C ± 2° C. On le verse sur un mélange eau-glace. On amène à pH10 par addition de soude. On essore, lave et sèche. On obtient un produit que l'on purifie selon les procédés classiques pour obtenir le produit recherché fondant à 159° C.

**EXEMPLE 2 : 8-amino 12-méthyl 10,11-dihydro 5,10-imino 5H-dibenzo[a,d][cycloheptène-11-one**

On introduit, sous agitation magnétique et atmosphère d'azote, 5,7 g de produit obtenu à l'exemple 1 dans 50 $cm^3$ d'acide chlorhydrique concentré. On ajoute à cette solution en 5 minutes, 16 g de chlorure stanneux hydraté à deux molécules d'eau. On maintient le mélange réactionnel sous agitation une heure. On refroidit au bain de glace. On alcalinise par de la lessive de soude. On extrait au chlorure de méthylène, lave à l'eau, sèche, filtre et distille à sec sous pression réduite. On obtient 6 g du produit brut recherché que l'on purifie par chromatographie en éluant par le mélange chlorure de méthylène méthanol (9-1). On obtient 4,7 g du produit recherché (F = 160° C).

**EXEMPLE 3 : 8-amino 12-méthyl 10,11-dihydro 5,10-imino 5H-dibenzo[a,d]cycloheptène.**

On chauffe à 115° C pendant 1 heure 30 minutes 4 g de produit obtenu à l'exemple 2, 40 $cm^3$ d'éthylène glycol et 2,4 $cm^3$ d'hydrate d'hydrazine. On ajoute 4 g de potasse en pastilles. On chauffe pendant 2 heures 30 minutes à 170≈175° C. On refroidit le mélange réactionnel et le verse dans l'eau. On extrait au chlorure de méthylène, lave à l'eau, sèche, filtre et amène à sec. On obtient un produit que l'on chromatographie sur silice en éluant par le mélange chlorure de méthylène-méthanol (9-1). On obtient 3,1 g de produit de rf = 0,25 que l'on reprend dans 100 $cm^3$ d'éther éthylique. On traite avec du charbon actif, filtre et amène à sec. On obtient après recristallisation dans l'éther isopropylique 2,7 g du produit recherché fondant à 102-104° C.

**EXEMPLE 4 : 8-nitro 11-oxo 10,11-dihydro 5,10-imino 5H-dibenzo[a,d]cycloheptène-12-carboxylate d'éthyle.**

On agite pendant 24 heures au reflux une solution renfermant 6 g de produit obtenu à l'exemple 1, 60 $cm^3$ de benzène et 12 $cm^3$ de chloroformiate d'éthyle. On distille à sec sous pression réduite, extrait le résidu à l'éther, lave à l'aide d'une solution d'acide chlorhydrique normale. On sèche, filtre et distille à sec sous pression réduite. On recristallise dans l'éther isopropylique et essore. On obtient 6,2 g de produit recherché fondant à 156° C.

**EXEMPLE 5 : 8-nitro 10,11-dihydro 5,10-imino 5H-dibenzo[a,d]-cycloheptène-11-one.**

On agite pendant 20 heures à la température ambiante une solution renfermant 20 g de produit préparé à l'exemple 4, 200 $cm^3$ d'éthanol et 40 $cm^3$ de lessive de soude. On refroidit à 10° C environ, acidifie à l'aide d'une solution d'acide chlorhydrique concentré. On distille à sec sous pression réduite. On ajoute de l'eau, alcalinise à l'aide d'une solution concentrée d'ammoniaque et extrait au chlorure de méthylène. On lave à l'eau, sèche, filtre et distille à sec sous pression réduite. On recristallise le produit obtenu dans l'éther éthylique. On obtient 13,6 g d'un produit fondant à 145° C.

**EXEMPLE 6 : 8-amino 10,11-dihydro 5,10-imino 5H-dibenzo[a,d]-cycloheptène-11-one.**

On ajoute 10 g de chlorure stanneux à une suspension renfermant 5,5 g du produit préparé à l'exemple 5, et 55 $cm^3$ d'acide chlorhydrique concentré. On chauffe la suspension obtenue à 50° C pendant 45 minutes. On refroidit, alcalinise à l'aide d'une solution d'ammoniaque concentrée. On extrait au chlorure de méthylène. On lave à l'eau, sèche sur sulfate de magnésium, filtre et distille à sec sous pression réduite. On obtient 4,9 g d'un produit que l'on purifie par recristallisation dans l'acétate d'éthyle. On obtient le produit recherché fondant à 135° C.

**EXEMPLE 7 : 8-amino 10,11-dihydro 5,10-imino 5H-dibenzo[a,d]-cycloheptène.**

On chauffe à 115° C pendant 45 minutes, une suspension renfermant 5,2 g de produit préparé à l'exemple 6, 52 $cm^3$ d'éthylène glycol et 2,6 $cm^3$ d'hydrate d'hydrazine. On ajoute 5,2 g de potasse en pastilles. On chauffe à 145° C pendant 2 heures 30 minutes. On refroidit, ajoute de l'eau, extrait au chlorure de méthylène et lave à l'eau. On sèche sur sulfate de magnésium, filtre et distille à sec sous pression réduite. On obtient 5 g d'un produit que l'on recristallise dans l'éther isopropylique. On obtient 4,6 g du produit recherché fondant à 98° C.

**EXEMPLE 8 : 8-formylamino N-méthyl 10,11-dihydro 5,10-imino 5H-dibenzo[a,d]cycloheptène.**

A une solution renfermant 5,1 g du produit préparé à l'exemple 3, on ajoute à 5° C en 30 minutes, 9 g de dicyclohexylcarbodiimide et 100 $cm^3$ d'acétate d'éthyle. On refroidit à 5° C environ et ajoute en 30 minutes environ, 2,06 $cm^3$ d'acide formique dans 25 $cm^3$ d'acétate d'éthyle. On maintient le mélange réactionnel pendant 1 heure à 5° C environ. On filtre, distille le filtrat à sec sous pression réduite. On obtient 9 g de produit recherché brut. On ajoute 100 $cm^3$ d'une solution diluée de bicarbonate de sodium. On extrait au chlorure de méthylène, lave à l'eau, sèche, filtre et distille à sec sous pression réduite. On obtient 5,5 g du produit recherché amorphe.

**EXEMPLE 9 : N-méthyl 8-méthylamino 10,11-dihydro 5,10-imino-5H-dibenzo[a,d]cycloheptène.**

A une suspension de 2,75 g d'hydrure de lithium-aluminium dans 140 $cm^3$ de tétrahydrofuranne anhydre, on ajoute vers 5° C une solution de 5,5 g de produit préparé à l'exemple 8, dans 55 $cm^3$ de tétrahydrofuranne anhydre. On agite 5 minutes vers 7° C puis pendant une heure à la température ambiante. On ajoute 100 $cm^3$ de tétrahydrofuranne, refroidit à 10-15° C, et ajoute 30 $cm^3$ de tétrahydrofuranne à 20 % d'eau et 5 $cm^3$ d'eau. On filtre, rince au chlorure de méthylène et distille à sec sous pression réduite. On obtient 5,8 g d'une huile que l'on additionne de 50 $cm^3$ d'eau, on extrait au chlorure de méthylène, lave à l'eau, sèche, filtre et distille à sec sous pression réduite. On recristallise le produit obtenu dans l'éther isopropylique et l'essore. On obtient 3,1 g de produit recherché fondant à 124° C.
De la même manière, on a préparé les produits suivants :

**EXEMPLE 10 : 8-nitro 10,11-dihydro 5,10-imino-5H-dibenzo[a,d]-cycloheptène**

**EXEMPLE 11 : 8-méthylamino 10,11-dihydro 5,10-imino-5H-dibenzo[a,d]-cycloheptène**

**EXEMPLE 12 : 8-acétylamino 10,11-dihydro 5,10-imino-5H-dibenzo[a,d]-cycloheptène**

**EXEMPLE 13 : [10,11-dihydro 5,10-imino-5H-dibenzo cycloheptèn-8-yl]carbamate de méthyle**

**EXEMPLE 14 : N[10,11-dihydro 5,10-imino-5H-dibenzo[a,d]-cycloheptèn-8-yl]N′,N′-diméthylurée**

**EXEMPLE 15 :**

On a réalisé une préparation pour injection de formule.

| | |
|---|---|
| Produit de l'exemple 7 | 20 mg |
| Excipient aqueux stérile qsp | 5 $cm^3$ |

**EXEMPLE 16 :**

On a réalisé une préparation pour injection de formule.

| | |
|---|---|
| Produit de l'exemple 7 | 10 mg |
| Excipient aqueux stérile | 5 $cm^3$ |

**EXEMPLE 17 :**

On a préparé des comprimés répondant à la formule :

| | |
|---|---|
| Produit de l'exemple 7 | 15 mg |
| Excipient q.s.p. pour un comprimé terminé à 250 mg<br>Détail de l'excipient : lactose, amidon, talc, stéarate de magnésium. | |

Effet antagoniste sur le récepteur NMDA du glutamate : libéra-tion de noradrenaline (NA) "in vitro"

Des tranches d'hippocampe (diamètre 2 mm, épaisseur 300 micromètres) de rat sont incubées dans du tampon Krebs bicarbonate en présence de $^3H$-NA. Après l'incorporation du neurotransmetteur, les tranches sont lavées puis perfusées (débit 1 ml/min) avec le même tampon. Les fractions sont recueillies toutes les 3 minutes et la radioactivité libérée dans le milieu est mesurée.

L'addition de 100 micromoles de NMDA (N-méthyl D-aspartate) dans le milieu de perfusion provoque une libération de $^3H$-NA. Cette réponse est bloquée par le produit, la concentration qui diminue de 50 % cette libération étant de 200 nanomoles.

Recherche de l'effet protecteur neuronal dans l'hippocampe.

L'essai a été réalisé chez le rat anesthésié. Une injection stéréotaxique d'acide quinolinique (5 microgrammes) est réalisée dans l'hippocampe droit. Le produit à étudier (ou le véhicule seul pour le lot d'animaux témoins) est administré par voie intrapéritonéale 30 minutes auparavant.

Une semaine plus tard, les cerveaux sont prélevés et coupés en tranches fines. Les tranches sont colorées au crésyl violet et photographiées.

Chez les animaux témoins, l'acide quinolinique détruit la couche $CA_1$ de l'hippocampe droit tandis que l'hippocampe gauche (contrôle) non injecté, est intacte.

L'hippocampe gauche des animaux n'a reçu aucun traitement. Chez les animaux ayant reçu une administration de 20 mg/kg du produit de l'exemple 7, par voie intrapéritonéale, la couche $CA_1$ de l'hippocampe droit est protégée donc identique à celle de l'hippocampe gauche qui n'a pas reçu d'injection d'acide quinolinique.

Activité anticonvulsivante chez la souris.

Technique.

L'essai est réalisé sur des lots de 10 souris. L'agent convulsivant est le pentétrazol, en solution aqueuse à la dose de 160 mg/kg s.c. sous un volume de 0,5 ml/20 g, administré 1 heure après le produit de l'exemple 7. Les animaux sont isolés et les convulsions toniques sont notées pendant 30 minutes.

La $DA_{50}$ ou dose qui diminue de 50 % le nombre de convulsions toniques entrainées par le pentétrazol est de 2,3 mg/kg.

**Revendications**

1) Les composés de formule (I) :

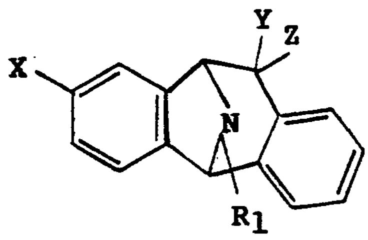

(I)

dans laquelle :
- X représente un groupe nitro ou un groupe $R_2R_3N$- dans lequel $R_2$ représente un atome d'hydrogène, un radical alkyle, alkényle ou alkynyle renfermant jusqu'à 8 atomes de carbone et $R_3$ représente un atome d'hydrogène, un radical alkyle, alkényle ou alkynyle renfermant jusqu'à 8 atomes de carbone, un radical arylalkyle renfermant jusqu'à 14 atomes de carbone, un radical $R_4\overset{\overset{O}{\|}}{C}$ - dans lequel $R_4$ représente un atome d'hydrogène ou un radical alkyle renfermant jusqu'à 8 atomes de carbone, un radical $-COOR_5$ dans lequel $R_5$ représente un radical alkyle renfermant jusqu'à 8 atomes de carbone, ou un radical $R_6R_7N\overset{\overset{O}{\|}}{C}$ -dans lequel $R_6$ et $R_7$ identiques ou différents, représentent un atome d'hydrogène ou un radical alkyle renfermant jusqu'à 8 atomes de carbone, à la condition que $R_2$ et $R_3$ ne représentent pas chacun un radical alkyle,
- Y et Z, ou bien représentent chacun un atome d'hydrogène,
ou bien forment ensemble avec l'atome de carbone auquel ils sont liés un groupe carbonyle et
- $R_1$ représente un atome d'hydrogène, un radical alkyle, alkényle ou alkynyle renfermant jusqu'à 8 atomes de carbone ou un radical $-COOR_8$ dans lequel $R_8$ représente un radical alkyle renfermant jusqu'à 8 atomes de carbone, ou un radical arylalkyle, éventuellement substitué, renfermant jusqu'à 14 atomes de carbone ainsi que leurs sels d'addition avec les acides organiques ou minéraux.

2) Les composés de formule (I) tels que définis à la revendi cation 1, dans lesquels X représente un groupe amino ainsi que leurs sels d'addition avec les acides organiques et minéraux.

3) Les composés de formule (I) tels que définis à la revendication 1 ou 2 dans lesquels Y et Z représentent chacun un atome d'hydrogène ainsi que leurs sels d'addition avec les acides organiques et minéraux.

4) Les composés de formule (I) tels que définis à l'une quelconque des revendications 1 à 3 dans lesquels $R_1$ représente un atome d'hydrogène ainsi que leurs sels d'addition avec les acides organiques ou minéraux.

5) Le composé de formule (I) tel que défini à la revendication 1 dont le nom suit :
Le 8-amino 10,11-dihydro 5,10-imino 5H-dibenzo[a,d]cycloheptène ainsi que ses sels d'addition avec les acides organiques ou minéraux.

6) A titre de médicaments, les composés de formule (I) tels que définis à l'une quelconque des revendications 1 à 4 ainsi que leurs sels d'addition avec les acides pharmaceutiquement acceptables.

7) A titre de médicament le composé de formule (I) défini à la revendication 5 ainsi que ses sels d'addition avec les acides organiques ou minéraux pharmaceutiquement acceptables.

8) Les compositions pharmaceutiques renfermant comme principe actif au moins un médicament défini à la revendication 6 ou 7.

9) Procédé de préparation des composés de formule (I) tels que définis à l'une quelconque des revendications 1 à 5, caractérisé en ce que l'on soumet un composé de formule (II) :

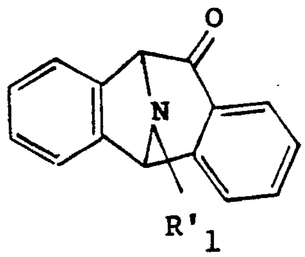

(II)

dans laquelle $R'_1$ représente un radical alkyle, alkényle ou alkynyle renfermant jusqu'à 8 atomes de carbone à l'action d'un agent de nitration pour obtenir le composé de formule ($I'_A$) correspondant :

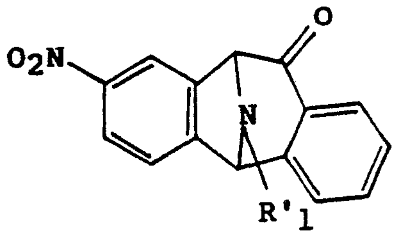

($I'_A$)

puis soumet le composé de formule ($I'_A$) ainsi obtenu à la totalité ou à une partie seulement des opérations suivantes dans un ordre quelconque :

- réduction sélective de la fonction nitro et éventuellement dans ce cas fonctionnalisation du radical amino obtenu,
- réduction sélective du groupe carbonyle,
- remplacement du radical $R'_1$ par un radical $-COOR_8$ et éventuellement dans ce cas, clivage du groupe $COOR_8$ obtenu conduisant au composé dans lequel $R'_1$ est un atome d'hydrogène,
- salification des produits de formule (I) ainsi obtenus.

10) Procédé selon la revendication 9, caractérisé en ce que l'on soumet le composé de formule ($I_A$) :

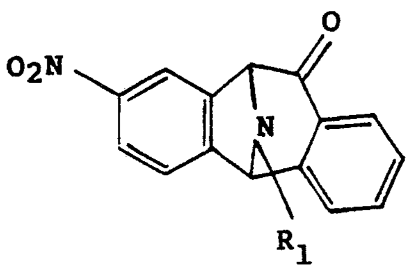

($I_A$)

dans laquelle $R_1$ conserve sa signification précédente, à l'action d'un agent de réduction sélective du radical nitro, pour obtenir le composé de formule ($I_B$) correspondant :

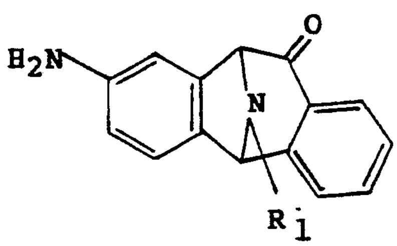

($I_B$)

que l'on soumet si désiré à l'action d'un agent de réduction sélective de la fonction cétone, pour obtenir le composé de formule ($I_C$) :

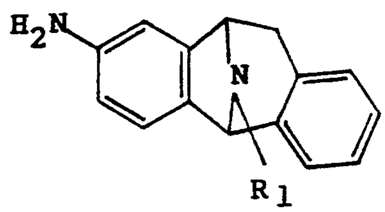

$(I_C)$

puis soumet si désiré les composés de formule (I) à l'action d'un acide pour en former les sels.

11) Procédé selon la revendication 9 caractérisé en ce que l'on soumet le composé de formule ($I''_A$) :

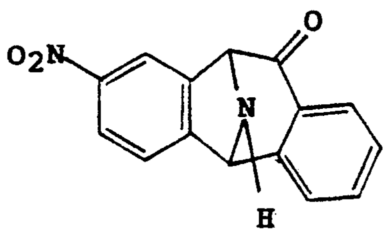

$(I''_A)$

c'est-à-dire le composé de formule ($I_A$) dans lequel $R_1$ est un atome d'hydrogène,
ou bien à l'action d'un agent capable d'introduire le radical $R''_1$ pour obtenir le composé de formule ($I'_B$) :

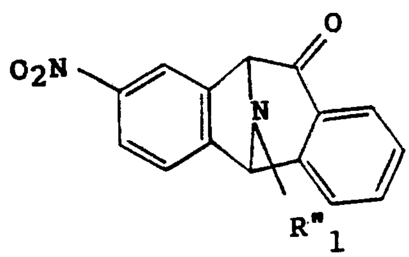

$(I'_B)$

dans lequel $R''_1$ représente un radical arylalkyle éventuellement substitué, renfermant jusqu'à 14 atomes de carbone ou un radical $-COOR_8$, $R_8$ conservant sa signification précédente, que l'on soumet, le cas échéant, à l'action d'un agent de réduction du groupement carbonyle, pour obtenir le composé de formule ($I'_C$) :

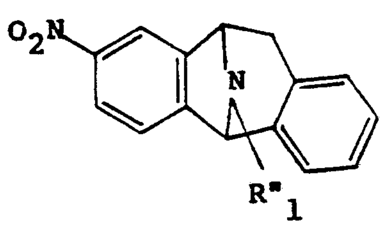

$(I'_C)$

ou bien à l'action d'un agent de réduction du groupement carbonyle pour obtenir le composé de formule générale ($I''_B$) :

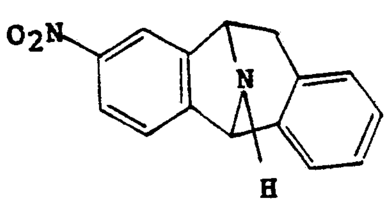

$(I''_B)$

que l'on soumet, le cas échéant, à l'action d'un agent capable d'introduire le radical $R''_1$, pour obtenir le composé de formule ($I'_C$) :

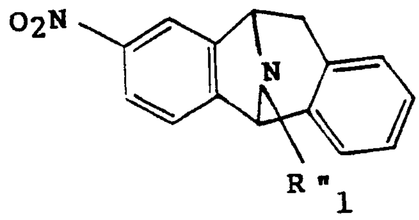

(I'C)

puis soumet, le cas échéant, le composé de formule (I'C) ainsi obtenu, à l'action d'un agent de réduction du groupement nitro, pour obtenir le composé de formule (I'D) :

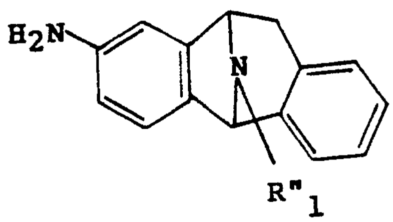

(I'D)

que l'on soumet, le cas échéant, à l'action d'un agent de fonctionnalisation du groupement amino pour obtenir le composé de formule (I'E) :

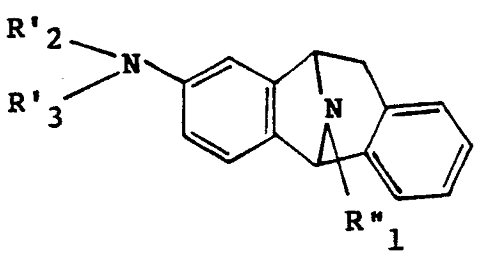

(I'E)

dans lequel $R'_2$ et $R'_3$ ont la même signification que $R_2$ et $R_3$ sans pouvoir représenter tous deux un atome d'hydrogène, que l'on soumet, le cas échéant, à l'action d'un agent de déprotection de l'amine $-NR''_1$ pour obtenir le composé de formule (I'F) :

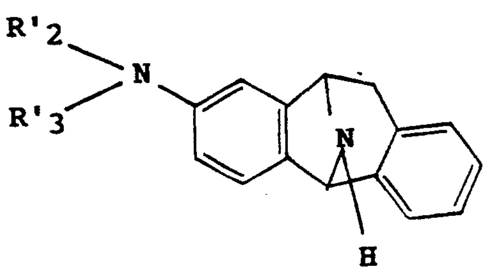

(I'F)

puis si désiré, soumet les composés de formule (I) ainsi obtenus à l'action d'un acide pour en former les sels.

Revendications pour les Etats contractants suivants: ES, GR

1.- Procédé pour préparer les composés de formule (I) :

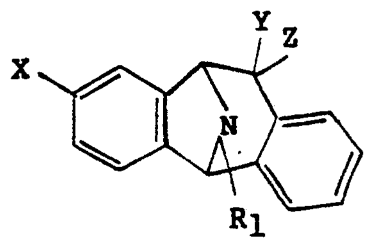

(I)

dans laquelle :

- X représente un groupe nitro ou un groupe $R_2R_3N$- dans lequel $R_2$ représente un atome d'hydrogène, un radical alkyle, alkényle ou alkynyle renfermant jusqu'à 8 atomes de carbone et $R_3$ représente un atome d'hydrogène, un radical alkyle, alkényle ou alkynyle renfermant jusqu'à 8 atomes de carbone, un radical arylalkyle renfermant jusqu'à 14 atomes de carbone, un radical $R_4\overset{\overset{O}{\|}}{C}$ - dans lequel $R_4$ représente un atome d'hydrogène ou un radical alkyle renfermant jusqu'à 8 atomes de carbone, un radical $-COOR_5$ dans lequel $R_5$ représente un radical alkyle renfermant jusqu'à 8 atomes de carbone, ou un radical $R_6R_7N\overset{\overset{O}{\|}}{C}$ -dans lequel $R_6$ et $R_7$ identiques ou différents, représentent un atome d'hydrogène ou un radical alkyle renfermant jusqu'à 8 atomes de carbone, à la condition que $R_2$ et $R_3$ ne représentent pas chacun un radical alkyle,
- Y et Z, ou bien représentent chacun un atome d'hydrogène,

ou bien forment ensemble avec l'atome de carbone auquel ils sont liés un groupe carbonyle et

- $R_1$ représente un atome d'hydrogène, un radical alkyle, alkényle ou alkynyle renfermant jusqu'à 8 atomes de carbone ou un radical $-COOR_8$ dans lequel $R_8$ représente un radical alkyle renfermant jusqu'à 8 atomes de carbone, ou un radical arylalkyle, éventuellement substitué, renfermant jusqu'à 14 atomes de carbone ainsi que leurs sels d'addition avec les acides organiques ou minéraux, caractérisé en ce que l'on soumet un composé de formule ($I'_A$) correspondant :

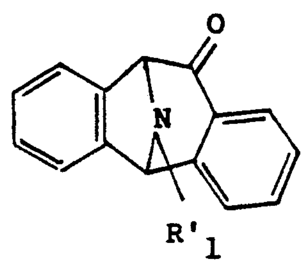

(II)

dans laquelle $R'_1$ représente un radical alkyle, alkényle ou alkynyle renfermant jusqu'à 8 atomes de carbone à l'action d'un agent de nitration pour obtenir le composé de formule ($I'_A$) correspondant :

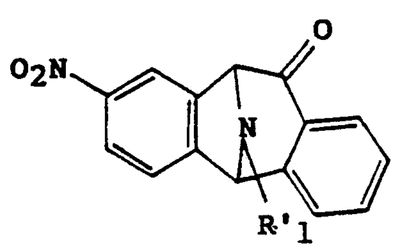

($I'_A$)

puis soumet le composé de formule ($I'_A$) ainsi obtenu à la totalité ou à une partie seulement des opérations suivantes dans un ordre quelconque :

- réduction sélective de la fonction nitro et éventuellement dans ce cas fonctionnalisation du radical amino obtenu,
- réduction sélective du groupe carbonyle,

- remplacement du radical $R'_1$ par un radical $-COOR_8$ et éventuellement dans ce cas, clivage du groupe $COOR_8$ obtenu conduisant au composé dans lequel $R'_1$ est un atome d'hydrogène,
- salification des produits de formule (I) ainsi obtenus.

2 ) Procédé selon la revendication 1, caractérisé en ce que l'on soumet le composé de formule ($I_A$) :

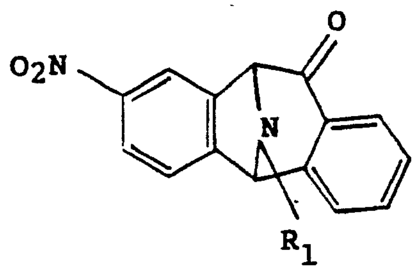

($I_A$)

dans laquelle $R_1$ conserve sa signification précédente, à l'action d'un agent de réduction sélective du radical nitro, pour obtenir le composé de formule ($I_B$) correspondant :

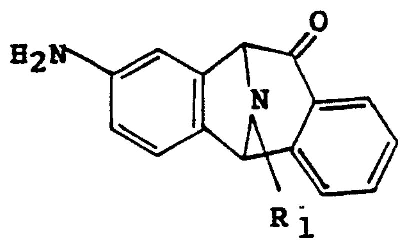

($I_B$)

que l'on soumet si désiré à l'action d'un agent de réduction sélective de la fonction cétone, pour obtenir le composé de formule ($I_C$) :

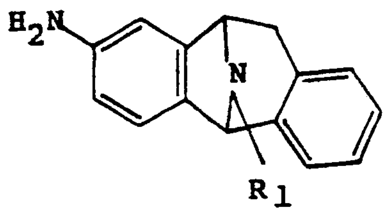

($I_C$)

puis soumet si désiré les composés de formule (I) à l'action d'un acide pour en former les sels.

3 ) Procédé selon la revendication 9 caractérisé en ce que l'on soumet le composé de formule ($I''_A$) :

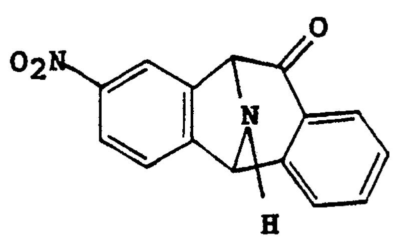

($I''_A$)

c'est-à-dire le composé de formule ($I_A$) dans lequel $R_1$ est un atome d'hydrogène,
ou bien à l'action d'un agent capable d'introduire le radical $R''_1$ pour obtenir le composé de formule ($I'_B$) :

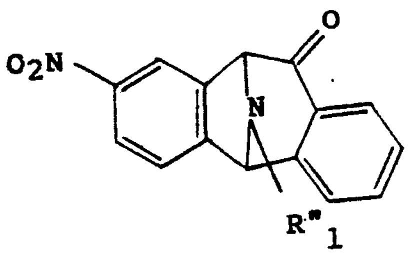

$(I'_B)$

dans lequel $R''_1$ représente un radical arylalkyle éventuellement substitué, renfermant jusqu'à 14 atomes de carbone ou un radical $-COOR_8$, $R_8$ conservant sa signification précédente, que l'on soumet, le cas échéant, à l'action d'un agent de réduction du groupement carbonyle, pour obtenir le composé de formule $(I'_C)$ :

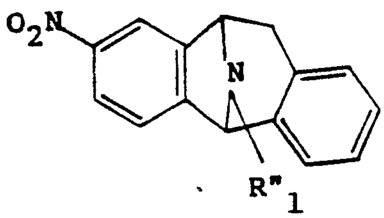

$(I'_C)$

ou bien à l'action d'un agent de réduction du groupement carbonyle pour obtenir le composé de formule générale $(I''_B)$ :

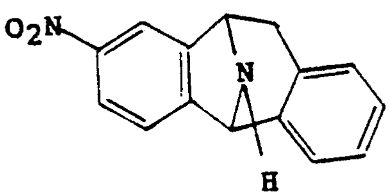

$(I''_B)$

que l'on soumet, le cas échéant, à l'action d'un agent capable d'introduire le radical $R''_1$, pour obtenir le composé de formule $(I'_C)$ :

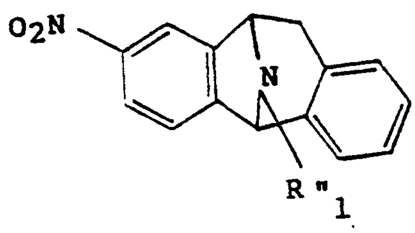

$(I'_C)$

puis soumet, le cas échéant, le composé de formule $(I'_C)$ ainsi obtenu, à l'action d'un agent de réduction du groupement nitro, pour obtenir le composé de formule $(I'_D)$ :

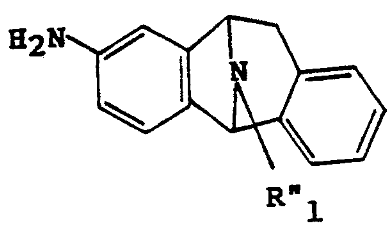

$(I'_D)$

que l'on soumet, le cas échéant, à l'action d'un agent de fonctionnalisation du groupement amino pour

obtenir le composé de formule ($I'_E$) :

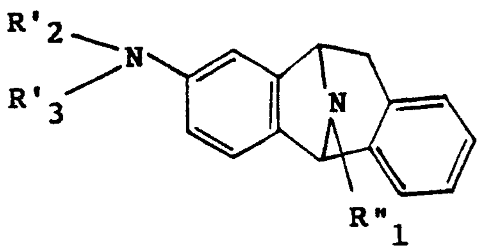

($I'_E$)

dans lequel $R'_2$ et $R'_3$ ont la même signification que $R_2$ et $R_3$ sans pouvoir représenter tous deux un atome d'hydrogène, que l'on soumet, le cas échéant, à l'action d'un agent de déprotection de l'amine $-NR''_1$ pour obtenir le composé de formule ($I'_F$) :

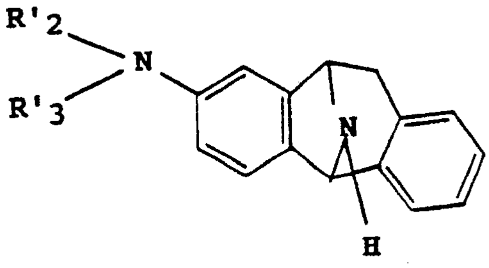

($I'_F$)

puis si désiré, soumet les composés de formule (I) ainsi obtenus à l'action d'un acide pour en former les sels.

4) Procédé selon la revendication 1, caractérisé en ce que l'on soumet un composé de formule (II) dans laquelle $R'_1$ représente un radical méthyle à l'action d'un agent de nitration puis à l'action d'un agent capable de remplacer $R'_1$ par un radical $COOR_8$ dans lequel $R_8$ a la signification précédente, puiq clive le groupe $-COOR_8$ et réduit sélectivement le radical nitro puis le groupe carbonyle afin d'obtenir le 8-amino 10,11-dihydro 5,10-imino 5H-dibenzo/a,d/ cycloheptène que l'on salifie si désiré.

Office européen des brevets

# RAPPORT DE RECHERCHE EUROPEENNE

Numero de la demande

EP 89 40 3081

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 170 862 (ROUSSEL-UCLAF)<br>* Revendications 1,6; page 5, lignes 13-20 *<br>----- | 1,6 | C 07 D 451/02<br>A 61 K 31/46 |
| | | | **DOMAINES TECHNIQUES RECHERCHES (Int. Cl.5)** |
| | | | C 07 D 451/00<br>A 61 K 31/00 |

Le présent rapport a été établi pour toutes les revendications

| Lieu de la recherche | Date d'achèvement de la recherche | Examinateur |
|---|---|---|
| LA HAYE | 05-02-1990 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)